# EUROPEAN PATENT APPLICATION

(11) **EP 2 372 590 A1**
(43) Date of publication of application: **05.10.2011**
(21) Application number: 10003571.6
(22) Date of filing: 31.03.2010
(51) Int. Cl.: G06F 19/00

(54) **Assembly station with component backtracking features**

(71) Applicant: F.Hoffmann-La Roche AG, 4070 Basel (CH); Roche Diagnostics GmbH, 68305 Mannheim (DE)
(72) Inventor: Besson, Marc, 3400 Burgdorf (CH); Probst, Roland, 3421 Lyssach (CH)
(74) Representative: Poredda, Andreas

(57) **Abstract**

The disclosure is directed towards a manual device assembly station for a device (10) including a plurality of components, the assembly station including:
a) a plurality of component-carriers (110), each component-carrier (110) being designed to carry a plurality of identical components, each component-carrier including a machine-readable code (125), the code (125) comprising backtracking data for the components carried by the corresponding component-carrier (110),
b) at least one code scanner (120), the code scanner (120) being designed to scan the codes carried by the component-carriers (110),
c) a plurality of trigger devices (115), each trigger device (115) being associated with a corresponding component-carrier (110), each trigger devices (115) being designed to generate a trigger signal upon occurrence of a scanning condition with respect to the corresponding component-carrier (110),
the trigger devices (115) being operatively coupled to the at least one code scanner (120) to activate the at least one code scanner (120) upon occurrence of the scanning condition such that the at least one code scanner (120) automatically scans the code of the component carrier (110) associated with the trigger device (115) from which the trigger signal was received,
d) a control computer (105), the control computer (105) being operatively coupled to the at least one code scanner (120), wherein the control computer (105) is configured
• to store an individual device identifier of a device (10) under assembly,
• to store backtracking data included in the scanned code (125) along with the individual device identifier.

The disclosure is further directed towards a method for generating backtracking data in a manual device assembly as well as to a use of such an assembly station and/or such a method for generating backtracking data in the assembly of diabetes therapy devices.

## Description

### Technical field

The present disclosure is related to the technical field of device assembly stations with component backtracking features and methods for generating backtracking data in assembly stations as well as the use of such assembly stations and methods. Embodiments in accordance with the present disclosure may especially be used in the context of the assembly of diabetes therapy devices and further medical equipment.

### Background

A state-of-the art therapy of diabetes mellitus is often based on Continuous Subcutaneous Insulin Infusion (CSII). In this therapy, a person with Diabetes (PwD) carries an insulin pump substantially continuous night and day. The insulin pump is a typically computer-controlled micro dosing device that administers small doses of an insulin formulation substantially continuous according to a time-variable personal basal administration profile via a subcutaneous cannula and additionally administers larger insulin boli on demand. Reference is made to WO 2003053498 A2 and W02000025844A1 for typical designs and characteristics of such pumps.

Since both over-dosing and under-dosing of drugs such as insulin are critical and may result in diverse medical complications, correct operation is of utmost importance. The devices therefore have to meet demanding regulatory and general quality standards. Component backtracking is a topic of particular importance in this context.

Backtracking allows securely identifying the lot or batch number, the manufacturer and/or further quality-related data of all components of a finished product. While many devices, such as insulin pumps, have an individual serial number, this is typically not the case for many or all of the components and sub-assemblies of such devices. Those components or sub-assemblies may be mass products, such as screws or loudspeakers, or may especially be designed for a specific type of device, such as injection molded plastic components, machine-tooled drive components, labels, or the like.

Besides insulin pumps, a similar situation occurs for other devices that are typically used by a PwD in diabetes therapy, such as continuous or non-continuous blood glucose measurement devices. All such devices that are especially designed and suited for the therapy of diabetes are in the following referred to as "diabetes therapy devices".

The final assembly of such devices may be performed in a manual assembly process where the devices are assembled by one or multiple operators form components and/or sub-assemblies. In following, the term "component" is meant to include both single components, such as screws, and sub-assemblies, such as gear boxes.

Devices may be assembled in a single assembly station or in a number of consecutive assembly stations. An assembly station may especially be adapted for a specific type of device or may be used for the assembly of different devices. Even in the first case, however, there is typically some variety, for example with respect to the housing color and/or the country of sale. The number of identical devices that are manufactured in sequence is typically low and may, for example, be in the range of 1 to 500 individual devices.

In a current assembly station, the components are typically provided in component-carriers, such as boxes or trays. Each component-carrier comprises a plurality of identical and unmarked components. In this context, the term "identical und unmarked" indicates that the components are of the same type and are not marked individually with a serial number, a batch number, or other data that may be used for backtracking purposes. Each component-carrier includes a machine-readable code, such as a barcode, with backtracking data, such as a batch or lot number of the components in the carrier, Each time an empty component-carrier is replaced by a new one, an operator uses a hand-held barcode scanner or the like to read-in the code from the component-carrier. The code is fed into a control computer, such as a computer-based manufacture control system, which stores the code, fully or in part, along with individual serial numbers of the devices under assembly. In this way, backtracking is in principle enabled for all components of an individual device.

A number of failures, however, is possible to occur in this procedure, for example by forgetting to read-in the code from a fresh component-carrier or by mistakenly interchanging component-carriers.

Three exemplary scenarios for the occurrence of failures are illustrated in the following. It is assumed, that different devices or device lots, require, in part, alternative components of different alternative component types, referred to as Ct I, Ct II. Those components are alternatives for a common genereric component type. For example, a common generic component type is a housing component of the device and the component types Ct I, Ct II are housing components of different colors or housing components carrying a labeling in alternative languages. For a specific device or device lot, component Type Ct I shall be used.

Case A: During the assembly of a device lot, the component-carrier with components of component type Ct I is emptied. The component-carrier is replaced by a new one of a different component batch. Reading the barcode however is forgotten. It is therefore assumed in the control computer that components belong to the previous component batch. Incorrect backtracking data will accordingly be stored for the subsequently assembled devices.

Case B: The component-carriers for both component types Ct I and Ct II are emptied and have accordingly to be replaced. The barcodes are scanned correctly from the component-carriers. The component-carriers, however, are mistakenly interchanged and placed at the wrong physical positions. For subsequently assembled devices, the component type Ct II will accordingly be used rather than component type Ct I. In addition, incorrect backtracking data will be stored for those devices.

Case C: It is now additionally assumed that more than one operator is working at the same assembly station: While this is typically avoided, it is well possible in specific situations. In a failure scenario, a first operator physically exchanges an empty component-carrier for component type Ct I by a new one. A second operator uses components from the new component-carrier before the code on the new component-carrier is scanned. Incorrect backtracking data will accordingly be stored for at least some subsequently assembled devices before the code is finally scanned.

### Summary

It is an objective of the present disclosure to provide manual assembly stations which include improved component backtracking features. The objective is achieved based on the insight that improvements are possible by providing trigger devices that automatically detect the occurrence of a scanning condition as a situation in which the code of a component-carrier should be scanned, followed by automatically scanning and storing the scanned code.

While the following description is mainly focused on the assembly of diabetes therapy devices, similar or comparable problems are present in other fields of the medical devices and hospital equipment industry, for example in the assembly of blood, urine or tissue analyzers as widely used in the field of medical analytics. The application is therefore not limited to this specific application. While the devices under assembly may be complete and finished devices, they may also be sub-units such as a complex drive system.

Embodiments of manual device assembly stations may include:
a) a plurality of component-carriers, each component-carrier being designed to carry a plurality of identical components, each component-carrier including a machine-readable code, the code comprising backtracking data for the components carried by the corresponding component-carrier,
b) at least one code scanner, the code scanner being designed to scan the codes carried by the component-carriers,
c) a plurality of trigger devices, each trigger device being associated with a corresponding component-carrier, each trigger devices being designed to generate a trigger signal upon occurrence of a scanning condition with respect to the corresponding component-carrier,
   the trigger devices being operatively coupled to the at least one code scanner to activate the at least one code scanner upon occurrence of the scanning condition such that the at least one code scanner automatically scans the code of the component carrier associated with the trigger device from which the trigger signal was received,
d) a control computer, the control computer being operatively coupled to the at least one code scanner, wherein the control computer is configured
   - to store an individual device identifier of a device under assembly,
   - to store backtracking data included in the scanned code along with the individual device identifier.

The individual device identifiers are typically a serial number of the individual devices under assembly.

The component-carriers may be of various types. They may, for example, include boxes comprising mass products or may be special-purpose carriers. The special-purpose carriers are typically trays or fixtures with cavities or the like which correspond to the shape of the components.

The trigger detectors and the at least one code scanner may be operatively coupled directly or may be operatively coupled via the control computer. In the latter case, the trigger devices are coupled to the control computer. The control computer is configured to receive a trigger signal and to activate the at least one code scanner upon reception of the trigger signal. A trigger device is provided for each component carrier in a one-to-one way.

The control computer may be linked to or be part of a computer-based overall manufacture control system which is further used for warehouse keeping, general quality recording, assembly job dispatching, and the like.

The assembly station may include further elements such as one or multiple fixtures for the devices to be assembled, manually and/or electrically and/or pneumatically powered tools, such as screw drivers, adhesive and/or lubricant dispensing units, or the like.

A scanning condition is a condition in which the code carried by an individual component-carrier shall be scanned to ensure correct storing of the backtracking data. A scanning condition may especially be picking of a component from a component carrier and/or the completed replacement of an empty component carrier by a new filled component carrier.

The assembly station may include a plurality of carrier receptacles, each carrier receptacle being configured to receive, during operation, corresponding component-carriers. The carrier receptacles may include wall-mounted holders for the component-carriers, cavities in a desk with the shape of the cavities matching the shape of the corresponding component-carriers, or the like. The component-carriers and the corresponding carrier receptacles may all be of the same type. In many cases, however, a variety of different general and special-purpose carriers and corresponding carrier receptacles will be present. The carrier receptacles especially ensure a defined physical position of the single component-carriers. In embodiments including dedicated carrier receptacles, the trigger devices may be integral with the carrier receptacles.

In some embodiments including dedicated carrier receptacles, the control computer is configured to store a receptacle matching list of component types and carrier receptacles. The control computer may especially be configured to generate an alert upon a code scanning indicating a component-carrier being received by a carrier receptacle deviating from the receptacle matching list. In this type of embodiments, potential mistakes resulting from an operator loading a carrier receptacle with a wrong component-carrier, that is, with a component-carrier not comprising the expected type of components, are avoided.

In some embodiments, a single code scanner may be provided which is used for scanning the codes from all component-carriers. In alternative embodiments, however, a plurality of code scanners is provided, wherein each code scanner is associated with a corresponding component-carrier in a one-to-one interrelation. This is typically the case for embodiments where the codes comprised by the component-carriers are barcodes and the code scanners are barcode scanners. Those barcode scanners are favorably permanently mounted such that they can read the barcodes from the corresponding component-carriers. In embodiments including dedicated carrier receptacles, the code scanners may be integral with the carrier receptacles.

An alternative technology are RFID tags on the component-carriers and corresponding RFID code readers as scanning devices.

Alternatively to a plurality of code scanners that are associated with corresponding individual component-carriers, one or multiple movable code scanners may be used. In this case, the one or multiple code scanners, e.g., barcode scanners, are mounted to a kinematic that is typically coupled to and controlled by the control computer. The kinematics allows to selectively positioning a code scanner such that it can selectively scan codes from a plurality of component-carriers. In an exemplary arrangement with a plurality of component-carriers being arranged in a matrix, a combination of a linear kinematics and a corresponding code scanner may be provided for each row or column of component-carriers.

In some embodiments, the trigger devices are, at least in part, picking detectors, the picking detectors being designed to generate a trigger signal upon an operator picking a component from the corresponding component-carrier.

For this kind of embodiment, the correct code is automatically scanned every time the operator picks a component for assembly in a device. In this way, confusions, mistakes and further error sources are largely avoided. A picking detector may be realized in a number of ways. For example, light barriers may be used as picking detectors which are interrupted by the hand or arm of the operator when picking a component. If the components are provided in individual fixtures, cavities or the like, individual sensors, for example micro switches, may be provided for each fixture, cavity or the like. This is typically the case for components of complex geometry and/or special-purpose components, such as injection-molded parts. In embodiments including dedicated carrier receptacles, the picking detectors may be integral with the carrier receptacles.

In some embodiments, the trigger devices are, at least in part, manually operated control elements. For this kind of embodiment, an operator operates a control element, for example a pushbutton, every time a component is picked from the corresponding component-carrier. As compared to automated picking-detectors, the use of pushbuttons or the like requires less hardware effort and only consumes little additional time. The achieved safety level is similar to the use of picking detectors, provided that the corresponding control element is operated for each picking of a component.

In some embodiments, the control computer is configured to generate an alert upon an operator picking a component deviating from a component list for a device under assembly. In this way, a typical source of assembly errors can be eliminated. Assembly errors resulting from picking wrong components may especially occur if a product is manufactured in a number of variants, colors, etc.

In some embodiments, the assembly station includes a plurality of picking indicators, each picking indicator being associated with a corresponding component-carrier and being controlled by the control computer to indicate the component-carrier from which a component shall be picked for a device under assembly. A picking indicator typically is an optical indicator, such as a light, which is activated to indicate that a component shall be picked from the corresponding component-carrier. The control computer may alternatively activate a number of indicators in parallel, thus indicating a number of component-carriers in parallel, or in sequence. A picking indicator is favorably deactivated after the corresponding picking operation is carried out.

Arrangements of component-carriers and computer-controlled picking indicators are known mainly from the fields of logistics and warehouse technology as "pick by light" arrangements. In embodiments including dedicated carrier receptacles, the picking indicators may be integral with the carrier receptacles.

The EP 2039461 A2 discloses an assembly station with a plurality of manually operated and guided electrically operated tools, such as electrically powered screw drivers, which may be automatically selectively activated for a specific assembly step and subsequently deactivated. A pick-by-light system setup may be used to indicate components that shall be used for the assembly in a correct assembly sequence. Instead of providing a plurality of optical picking indicators, alternative approaches may be used. An operator working at the assembly station may, for example, carry a head set and receive computer-generated voice information with respect to the component-carriers from which components shall be picked, as known in the logistics and warehouse technology as "pick by voice".

In some embodiments including a component list, the control computer is configured to select a component list for a device under assembly from at least two alternative component lists, the at least two alternative component list including at least two alternative component types of a common generic component type, wherein components of the alternative components types are, during operation, provided in parallel in corresponding separate component-carriers in the assembly station. For this type of embodiment, the assembly station can be used for the assembly of different device variants with components for the different variants being provided in parallel

The device variants may, for example, differ with respect to a housing color and/or with respect to a country-specific labeling, such a country specific list of device error codes and/or emergency contact information. The components of different component types may belong to the same generic component type as described above.

Alternatively to selecting a component list from two or multiple component lists, the control computer may be configured to receive the appropriate component list for a single device or a device lot prior to their assembly.

In some embodiments, the trigger devices are, at least in part, exchange detectors. The exchange detectors are arranged to generate a trigger signal upon the exchange of a corresponding component-carrier. A component-carrier is typically removed by the operator and replaced by a new one when it is empty. The removed component-carrier may be discarded or refilled.

In this type of embodiment, a trigger signal may be generated and a scanning of the code may be carried out only when a component-carrier is exchanged rather than for each picking operation. An exchange detector may be, for example realized by an electrical switch that is opened when no component-carrier is present at a predefined position, e.g. in a carrier receptacle, while it is closed if a component-carrier is present, or vice versa. Alternatively, the exchange detector is realized as light barrier, for example as reflected-light barrier as capacitive sensor, a scale that determines the weight of a component carrier, or the like. The trigger signal is favorably only generated when the exchange is finished, that is, when the new component-carrier is in place.

It is a further objective of the present disclosure to provide improved methods for generating backtracking data in a manual device assembly.

Embodiments of the methods may include:
a) detecting, by a trigger device, the occurrence of a scanning condition, thus generating a trigger signal,
   wherein the trigger device belongs to a plurality of trigger devices, each trigger device being associated with a corresponding component-carrier,
   wherein each component-carrier comprises a plurality of identical components and includes a corresponding machine-readable code, the code including backtracking data for the components carried by the corresponding component-carrier,
b) scanning, following the generation of the trigger signal, the code on the corresponding component-carrier, and
c) storing backtracking data included in the code along with an individual device identifier of a device under assembly.

In some embodiment, the method includes indicating the component-carrier from which a component shall be picked in accordance with a component list for a device under assembly.

In some embodiments, the method includes selecting a component list for a device under assembly, at least in part, from at least two alternative component lists, the at least two alternative component list including at least two alternative component types of a common generic component type, wherein components of the alternative components types are provided in parallel in corresponding separate component-carriers.

In some embodiments, the method is a method for generating backtracking data in an assembly of diabetes therapy devices, in particular of insulin pumps.

It is a further objective of the present disclosure to enable the use of a manual assembly station and/or a method for generating backtracking data in the assembly of diabetes therapy devices, in particular in the assembly of insulin pumps.

It shall be noted that method steps that are disclosed in conjunction with structural features may be used for detailing method claims as well as use claims which are based on other portions of the description. In the same manner structural features that are disclosed in conjunction with methods or uses may be used for detailing structural claims.

### Detailed description of exemplary embodiments

In the following, exemplary embodiments of an assembly station in accordance with the present disclosure are explained in more detail with reference to the figures.
Figure 1 shows an exemplary assembly station in a schematic structural view.
Figure 2 shows an exemplary flow of the major operational steps for a device assembly in an assembly station according to Figure 1.

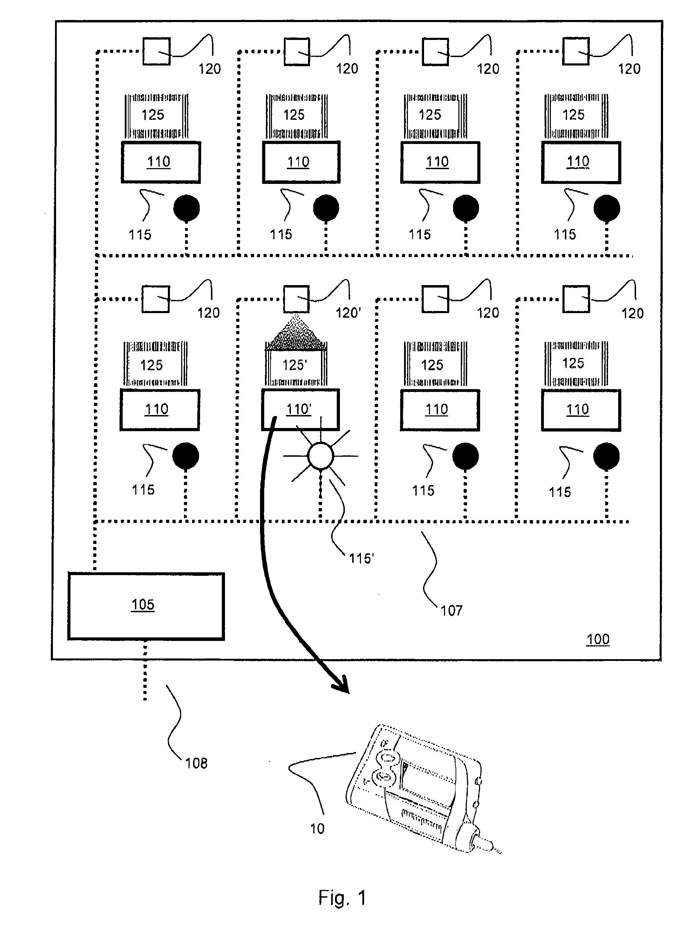

Figure 1 shows an exemplary assembly station 100 in a schematic structural view. The device to be assembled is referred to by reference number 10.

The assembly station 100 comprises a control computer 105 which may be a dedicated computer system or may be part of a larger IT infrastructure system, such as the computer network of a production facility. The assembly station 100 comprises a number of component-carriers 110 which may, for example, be boxes or trays and may be of the same or different types. Each component-carrier has a barcode 125 with backtracking data for the components in the corresponding component-carrier 110. Barcode scanners 120 are provided for scanning the barcodes 125. A user interface 115 is provided for each component-carrier 110. The user interfaces 115 are exemplarily realized as manually operated pushbuttons, the pushbuttons serving as trigger devices, with integrated optical indicators, the indicator serving as picking indicators. Instead of manually operated pushbuttons, light-barrier or kinds of automated picking-detectors may be provided for some of all of the component-carriers 110.

The control computer 105, the user interfaces 115 and the barcode scanners 120 are operatively coupled via a communication network 107. The communication network 107 may be a wired special-purpose network, a wired general-purpose network, such as an Ethernet or CAN network, a wireless network, or any suited combination. The control computer 105 is further connected with the overall IT infrastructure of the assembly facility via network 108.

In Figure 1, reference numbers with apostrophe, such as component-carrier 110', refer to elements that are currently selected or activated for picking a component while the reference numbers without apostrophe may refer to elements of the assembly station 100 in general and/or to non-activated elements.

In Figure 1, the optical indicator of the user interface 115' is shown as activated, indicating that a component shall be picked from component-carrier 110' for assembly. When picking a component from component-carrier 110', the operator operates the pushbutton of user interface 115'. The control computer 105 triggers the barcode scanner 120' to scan the corresponding barcode 125' and stores the backtracking data along with a serial number of device 10.

Instead of providing pushbuttons in the user interfaces 115, automatic picking-detectors, such as light barriers, may be used without deviating from the overall setup.

In Figure 1, no carrier receptacles are explicitly referenced. They may, however, be present and may be made by each combination of a user interface 115 and a barcode scanner 120 in combination with a holder, fixture, stand, or the like for the component-carrier 110 with barcode 125. Alternatively, the user interfaces 115 and the barcode scanners 120 may be directly removable attached to the corresponding component-carriers 110, e.g., via corresponding clamp.

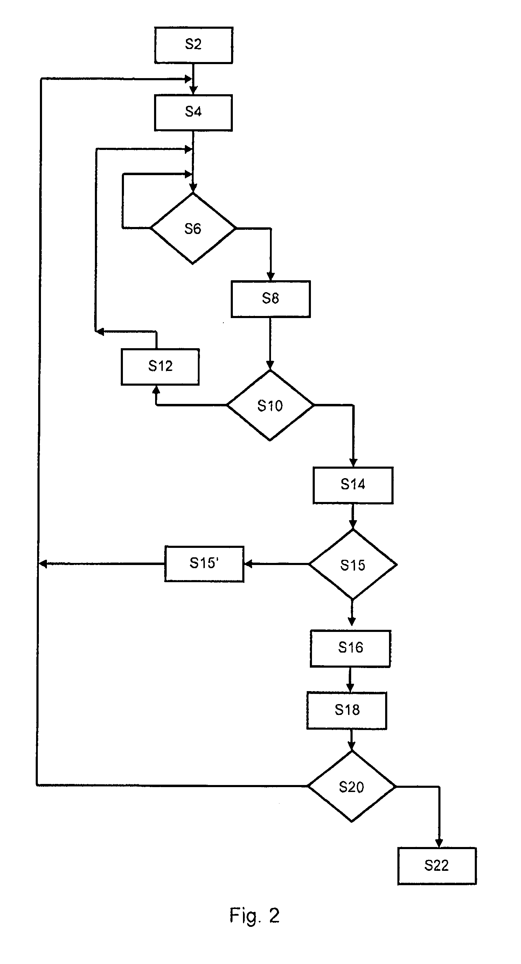

Some of the component-carriers 110 comprise alternative components of a common generic component type. Accordingly, only components from some of the component-carriers 110 have to be used for the assembly of an individual device 10. Figure 2 schematically shows the major operational steps for a device assembly on assembly station 100 as illustrated by Figure 1. In order to best focus on the specific aspects of the present disclosure, Figure 2 does not reflect all detailed steps which may typically occur in the assembly process and are known for a person skilled in the art.

In step S2, basic data of the device 10 to be assembled are loaded, via the network 108, into the control computer 105, in particular an individual serial number of device 10 and optionally further data such as a lot number of a device lot, a customer-identifier, the date of the assembly, the name of the operator carrying out the assembly, or the like. Along with those data, a component list of the components of device 10 is loaded into the control computer 105. Alternatively, those data may, completely or in part, already be present in the control computer 105. The control computer 105 further stores or receives a matching list of component types and carrier receptacles as described above in the general description of the invention.

In step S4, the control computer activates the picking-indicator for a first component to be assembled in accordance with the component list and the receptacle matching list. In step S6, the control computer 105 waits for the reception of a trigger signal, upon reception of which the operational flow proceeds with step S8. In step S8, the control computer 105 checks whether the trigger signal that was received in Step S6 originates from the correct trigger device, that is, from the trigger device associated with the component-carrier 110' that was indicated for picking. If this is not the case, an alert is generated in step S12 which has to be confirmed by the operator. In this case, the operator picked a component from a wrong component-carrier. After the confirmation, the operational flow returns to waiting for a trigger signal from the correct trigger device in step S6. The mistakenly picked component is typically returned to its component-carrier 110 by the operator.

If it is confirmed in S10 that the picked component was picked from the correct component-carrier 110', the corresponding barcode scanner 120' is activated and the barcode 125' of the component-carrier 110' is scanned in Step S14.

In following step 15, it is assessed whether the indicated component-carrier comprises the correct type of components. For this purpose, the component type as indicated by the barcode 125' on component-carrier 110' is compared with an expected component-type according to the matching list of component types and carrier receptacles. A mismatching may especially occur if a user has mistakenly misplaced a component-carrier. In this case, the operational flow proceeds with step S15' where an alert is generated and the mismatching is rectified by an operator. Subsequently, the assembly is continued.

In step S16, the barcode, or at least the backtracking information included in the barcode is stored to the further data of device 10 in the control computer 105. Following step S16, further steps that are specific for the assembly of a component may be carried out, such as temporarily activating a electric screw-driver, a adhesive dispenser, or the like in optional step S18.

In step S20, it is determined if the assembled component was the last component to be used in the assembly of the device 10. If this is not the case, that is, if further components have to be picked and assembled, the operational flow proceeds with step S4 in order to indicate the next following component to be used in the assembly. Since some of the component-carriers 110 may comprise alternative components, no components may be picked form a number of component-carriers 110.

If the component was the last component to be used in the assembly, the assembly of device 10 is finished and the operational flow proceeds with step S20 where the assembly of device 10' is marked as completed in the control computer in step S22.

It has to be understood that Figure 2 mainly reflects those operational steps that are carried out under control of the control computer 105. Those steps that are typically carried out manually, such as picking components from the component-carrier, actually assembling the components, putting the assembled device 10 into a carrier for completely assembled devices, manually or automated inspection steps, and the like, are not reflected but are typically present in the context of the overall assembly procedure.

It has further to be understood that the exemplary assembly station as illustrated in Figure 1 and the operational flow as illustrated in Figure 2 may be varied in diverse ways. In particular, the order of some steps of the operational flow may be altered and/or additional steps may be carried out. For example, the safety checks as reflected by step S6 and S10, S12, and S15, S15' may be omitted or carried out in a different way.

## Claims

1. Manual device assembly station for a device including a plurality of components, the assembly station including:
a) a plurality of component-carriers (110), each component-carrier (110) being designed to carry a plurality of identical components, each component-carrier including a machine-readable code (125), the code (125) comprising backtracking data for the components carried by the corresponding component-carrier (110),
b) at least one code scanner (120), the code scanner (120) being designed to scan the codes carried by the component-carriers (110),
c) a plurality of trigger devices (115), each trigger device (115) being associated with a corresponding component-carrier (110), each trigger devices (115) being designed to generate a trigger signal upon occurrence of a scanning condition with respect to the corresponding component-carrier (110),
the trigger devices (115) being operatively coupled to the at least one code scanner (120) to activate the at least one code scanner (120) upon occurrence of the scanning condition such that the at least one code scanner (120) automatically scans the code of the component carrier (110) associated with the trigger device (115) from which the trigger signal was received,
d) a control computer (105), the control computer (105) being operatively coupled to the at least one code scanner (120), wherein the control computer (105) is configured
• to store an individual device identifier of a device (10) under assembly,
• to store backtracking data included in the scanned code (125) along with the individual device identifier.

2. Assembly station (100) according to Claim 1, including a plurality of carrier receptacles, each carrier receptacle being configured to receive, during operation, corresponding component-carriers (115).

3. Assembly station (100) according to Claim 2, wherein the control computer (105) is configured to store a receptacle matching list of component types and carrier receptacles.

4. Assembly station (100) according to any of the preceding claims, wherein the at least one code scanner (120) comprises a plurality of code scanners (120), each code scanner (120) being associated with a corresponding component-carrier (110).

5. Assembly station (100) according to any of the preceding claims, wherein the trigger devices are, at least in part, manually operated control elements (115).

6. Assembly station (100) according to any of the preceding claims, wherein the control computer (105) is configured to generate an alert upon an operator picking a component deviating from a component list for a device under assembly,

7. Assembly station (100) according to any of the preceding claims, wherein the assembly station (100) includes a plurality of picking indicators (115), each picking indicator (115) being associated with a corresponding component-carrier (110) and being controlled by the control computer (105) to indicate the component-carrier (110) from which a component shall be picked for a device under assembly.

8. Assembly station according to any of the preceding claims, wherein the control computer is configured to select a component list for a device (10) under assembly, at least in part, from at least two alternative component lists, the at least two alternative component list including at least two alternative component types of a common generic component type, wherein components of the alternative components types are, during operation, provided in parallel in corresponding separate component-carriers (110) in the assembly station (100).

9. Method for generating backtracking data in a manual device assembly, the method including:
a) detecting, by a trigger device (115'), the occurrence of a scanning condition, thus generating a trigger signal,
wherein the trigger device (115') belongs to a plurality of trigger devices (115), each trigger device (115) being associated with a corresponding component-carrier (110'),
wherein each component-carrier (110) comprises a plurality of identical components and includes a corresponding machine-readable code (125), the code (125) including backtracking data for the components carried by the corresponding component-carrier (110),
b) scanning, following the generation of the trigger signal, the code (125) on the corresponding component-carrier (110), and
c) storing backtracking data included in the code along with an individual device identifier of a device (10) under assembly.

10. Method according to Claim 9, including indicating the component-carrier (110) from which a component shall be picked in accordance with a component list for a device (10) under assembly.

11. Method according to Claim 9 or Claim 10, including selecting a component list for a device (10) under assembly, at least in part, from at least two alternative component lists, the at least two alternative component list including at least two alternative component types of a common generic component type, wherein components of the alternative components types are provided parallel in corresponding separate component-carriers (110).

12. Method according any of Claim 9 to 11 for generating backtracking data in an assembly of diabetes therapy devices, in particular of insulin pumps.

13. Use of a manual device assembly station (100) according to either of Claim1 to Claim 8 and/or use of a method for generating backtracking data in accordance with Claim 12 in the assembly of a diabetes therapy devices (10), in particular in the assembly of insulin pumps.

14. Use according to Claim 13, wherein at least one of the components of a device (10) under assembly is selected as either of at least two alternative component types of a common generic component type, wherein components of the alternative components types are provided in parallel in corresponding separate component-carriers (110).

15. Use according to Claim 14, wherein the at least two alternative component types carry alternative user-readable information and/or have alternative colors.
